# EUROPEAN PATENT SPECIFICATION

(11) **EP 0 532 326 B1**
(45) Date of publication and mention of the grant of the patent: **17.04.1996**
(21) Application number: 92308246.5
(22) Date of filing: 10.09.1992
(51) Int. Cl.: B01J 31/18, C07D 487/22, C07C 29/50

(54) **Nitrated metalloporphyrins as catalysts for alkane oxidation**
Nitrierten Metalloporphyrine als Katalysatoren bei der Oxydierung von Alkanen
Métalloporphyrines nitrées comme catalyseurs pour l'oxydation d'alcanes

(30) Priority: 12.09.1991 US 758147; 02.06.1992 US 892106
(43) Date of publication of application: 17.03.1993
(73) Proprietor: SUN COMPANY, INC. (R&M), Philadelphia, PA 19103-1699 (US)
(72) Inventor: Ellis, Paul E., Jr., Downingtown, PA 19335 (US); Lyons, James E., Wallingford, PA 19086 (US)
(74) Representative: Lewin, John Harvey

(56) References cited:
- WO-A-88/07988
- US-A- 4 746 735
- US-A- 4 895 682
- CATALYSIS LETTERS vol. 8, no. 1, January 1991, BASEL . SWITZERLAND pages 45 - 52 E.LYONS E.ELLIS 'SELECTIVE LOW TEMPERATURE HYDROXYLATION OF ISOBUTANE BY MOLECULAR OXYGEN CATALYTED BY AN IRON PERHALOPORPHYRIN COMPLEX'

## Description

The Government of the United States of America has rights in this invention pursuant to Cooperative Agreement No. DE-FC21-90MC26029 awarded by the U. S. Department of Energy.

### BACKGROUND OF THE INVENTION

The invention relates to oxidation of alkanes using metalloporphyrins as catalysts, and more particularly to such processes in which nitro groups have been substituted for hydrogen in the porphyrin ligand.

The use of metalloporphyrins as catalysts for the oxidation of hydrocarbons with air in the liquid phase has been shown n our U. S. Patents 4,895,680 and 4,895,682 with the further finding that halogenation of the porphyrin ring led to even more active and stable catalysts (our U. S. Patents 4,900,871 and 4,895,682 and pending application Serial No. 568,118 filed August 16, 1990 = EP-A-0 471 561). Since these discoveries we have been able to correlate increased electron withdrawal from halogenation of the porphyrin ring to increased catalytic air oxidation activity. J. E. Lyons and P. E. Ellis, Jr., Catalysis Letters 8, 45 (1991).

It is also known that other functional groups besides halogens can lead to electron withdrawal in porphyrins. For example, L. C. Gong and D. Dolphin in Can. J . Chem., 63, 401 (1985) found that successive nitration at the meso positions of Zn(octaethylporphine) eventually giving Zn(mesotetranitrooctaethylporphine) led to more easily reduced porphyrins, evidence for electron withdrawal from the ring. Other workers such as Catalano, et.al. in J. Chem. Soc., 1535 (1984) have been able to nitrate the beta or pyrrolic positions in various metal tetraphenylporphyrins.

### DESCRIPTION OF THE INVENTION

We have now found that nitrated metalloporphyrins and nitrated/halogenated metalloporphyrins have utility as catalysts for the oxidation with oxygen-containing gas of alkanes such as methane, ethane, propane and butanes.

The process of the invention comprises contacting alkane with oxygen-containing gas in the presence of a metalloporphyrin in which 12.5 to 100 percent of the hydrogen atoms in the porphyrin ring have been replaced with nitro groups.

Preferably, the metal in the catalyst is selected from the group consisting of iron, chromium, manganese, ruthenium, cobalt or copper. Other metals known for making metalloporphyrins may be used, but in general such other metalloporphyrins are less active than metalloporphyrins containing the preferred metals above.

In one embodiment of the invention, the metalloporphyrin is substituted both with nitro groups and with halogen atoms. In such cases, preferably, 4 to 28 percent of the hydrogen atoms in the porphyrin ring have been replaced with nitro groups and 0 to 72 percent of the hydrogen atoms in the porphyrin ring have been replaced with halogen atoms. For example, in a porphyrin substituted with 20 fluorine atoms and 8 nitro groups, about 28 percent of the hydrogen atoms have been replaced with nitro groups and about 72 percent of the hydrogen atoms have been replaced with halogen atoms.

In one embodiment, 1 to 8 of the pyrrolic hydrogens in the porphyrin ring have been replaced with nitro groups. In a further embodiment, remaining hydrogens in the porphyrin ring have been replaced with halogen.

Preferably all of the hydrogen atoms have been replaced either with nitro groups or halogen atoms, but this is not essential.

Specific catalysts useful according to the invention include nitrated meso-perfluorinatedalkylporphyrin, nitrated iron tetrakispentafluorylphenylporphyrin and metallomesotetranitroporphine.

The catalysts used in the invention are particularly effective in the oxidation of alkanes and alkenes, including cycloalkanes and cycloalkenes, substituted alkanes and alkenes. The starting materials include straight and branched-chain compounds having from about 1 to 2 carbon atoms, preferably 1 to 10 carbon atoms, such as methane, ethane, propane, n-butane, isobutane, n-pentane, n-hexane, 2-methylpentane, 3-methylpentane, heptane, 2-methylheptane, 3-methylheptane and the corresponding alkene forms. as well as cycloalkanes and cycloalkenes having from 5 to 20 carbon atoms, preferably 5 to 10 carbon atoms, such as cyclopentane, cyclohexane, cycloheptane, cyclooctane and the corresponding alkene forms.

The oxidation, which may be carried out in a generally known manner, is desirably conducted in the liquid phase, although this is not critical, using such organic solvents as benzene, acetic acid, acetonitrile, methyl acetate, or like solvents which are inert to the conditions of the reactions, or in a neat solution of the hydrocarbon if it is liquid, and under pressures ranging from 1.03 to 103 bar (15 to 1500 psig), preferably 2.07 to 51.7 bar (30 to 750 psig), at temperature of from 25 to 250° C., more preferably 70 to 180° C. Depending upon whether the hydrocarbon to be oxidized is a solid, liquid or gas, it is dissolved in or bubbled through the solvent, together with air or oxygen, in the presence of the catalyst used according to the invention, for periods of time sufficient to yield the desired oxidation product, generally from 0.5 to 100 hours, and more preferably from 1 to 10 hours.

The choice of solvent, while not critical, can have an effect on the rates and selectivities obtained and should be carefully selected in order to optimize the desired results. For example, it has been found that solvents such as acetonitrile and acetic acid are often very effective for the oxidation of alkanes to form oxygen-containing compounds, whereas reactions carried out in solvents such as methyl acetate or benzene may occur more slowly. Thus, by routine experimentation, the optimum solvent for the particular process can be readily determined.

The ratios of the various reactants may vary widely, and are not critical. For example, the amount of catalyst employed can range from 10⁻⁶ to 10⁻³moles per mole of hydrocarbon such as alkane, and more preferably from about 10⁻⁵ to 10⁻⁴ mole of catalyst per mole of hydrocarbon, although other amounts are not precluded; while the amount of oxygen relative to the hydrocarbon starting material may also vary widely, generally 10⁻²to 10² moles of oxygen per mole of hydrocarbon. Care should be taken since some of the ratios fall within explosive limits. -As a group, the catalysts are almost always soluble unless used in large excess. Thus, as a rule, the reactions are generally carried out homogeneously.

The catalysts used in the process of the invention may be made by any suitable method, of which the following are examples.

### EXAMPLE 1

Iron tetrakispentafluorophenylporphine chloride is reacted with nitrogen dioxide (1-8) equivalents in methylene chloride or benzene leading to varying amounts of nitration at the beta positions on the ring according to the severity of the reaction conditions. Beta-positions left unnitrated are subsequently halogenated using normal chlorination, bromination or fluorination techniques. The general structure for the products is: 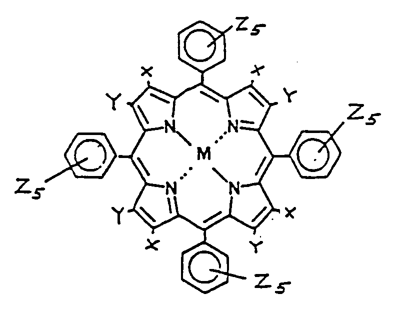
where M is Fe, Cr, Mn, Ru, Co or Cu, X is nitro, Y is nitro or Cl or Br or F and Z is H or Cl or Br or F.

### EXAMPLE 2

Zn(porphine) is reacted with nitrogen dioxide in methylene chloride to produce Zn(mesotetranitroporphine). The zinc is removed by acid treatment and Fe or other transition metal, M, is inserted by the usual method of ferrous chloride or MCl₂ in dimethylformamide. The beta or pyrrolic hydrogens can be further nitrated or halogenated as desired. The general formula is: 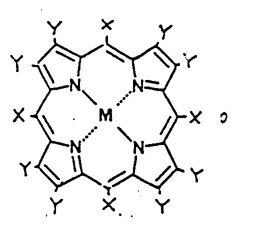
where M is Fe, Cr, Mn, Ru, Cu or Co, X is nitro, and Y is nitro, Cl, F, Br or any combination thereof.

### EXAMPLE 3

Meso-perfluorinated alkyl porphyrins, made as disclosed in our copending application Serial No. 568,118 filed August 16, 1990, (= EP-A-0 471 561), can be nitrated in the beta or pyrrolic positions using nitrogen dioxide in methylene dichloride or nitric acid/sulfuric acid nitrating solutions. The general structure is: 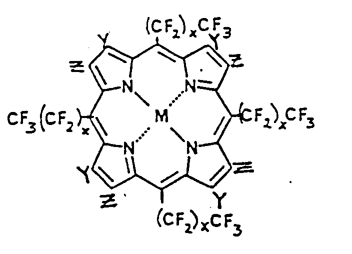
where M is Fe, Cr, Mn, Ru, Cu or Co, X is 0-6, and Y is nitro and Z is NO₂ or Cl or Br or F.

The invention will be further disclosed with reference to the following examples.

### EXAMPLE 4

The catalyst prepared as described in Example 1 is used as a catalyst for the oxidation of isobutane to t-butyl alcohol in the following manner. Isobutane (6-7 grams) is dissolved in 25 ml of benzene containing the catalyst, and air is added to a pressure of 100 psi. Oxidation is carried out at a temperature of 60° C. for six hours. Gaseous and liquid products are analyzed by gas chromatography and mass spectrometry. Catalyst activity is expressed as "catalyst turnovers", i.e., moles of oxygen consumed/mole of catalyst. Selectivity is the moles of TBA per mole of liquid product. Higher numbers of catalyst turnovers and/or greater selectivity are obtained with the catalyst of the invention as compared with otherwise similar catalyst which has not been substituted with nitro groups. Similar results are obtained when the catalysts of Examples 2 and 3 above are used as alkane oxidation catalysts.

### NEW COMPOUNDS

In this embodiment, the invention relates to metalloporphyrins useful as catalysts for the oxidation of alkanes, and more particularly to metalloporphyrins containing nitro groups on the porphyrin ring.

Nitro-substituted metalloporphyrins are known in the art. R. Bonnett et al, J. Chem. Soc. , 30, 2791-2798 (1965) disclose a nickel complex of α-nitrooctaethylporphyrin, and also uncomplexed α,β,γ-trinitrooctaethylporphyrin. E. C. Johnson et al, Tetr. Lett. 26, 2197 (1976) disclose a magnesium complex of 5,10,15,20-tetranitrooctaethyl - porphine. L. C. Gong et al, Com. J. Chem., 63, 401-405 (1985), disclose zinc complexes of mono-, di-, tri- and tetra-nitrooctaethylporphyrins. J. E. Baldwin et al, Tetrahedron, 38, 685 (1982), disclose a zinc complex of dinitrotetraphenylporphine. M. Catalano et al, J. Chem, Soc. Chem. Commun., 1535-1536 (1984) disclose nickel, palladium, copper, cobalt, iron, magnesium and zinc complexes of betanitromesotetraarylporphyrins containing in some instances one and in some instances two nitro groups.

The complexes of the Bonnett, Johnson and Gong disclosures above have octaethyl groups at the β-pyrrolic positions of the porphyrin ring and nitro groups at the meso positions of the ring. The complexes of the Baldwin and Catalano disclosures have aryl, e.g. phenyl, groups at the meso positions of the ring, and nitro groups at one or two β-pyrrolic positions of the ring.

U. S. Patent 5,077,394, issued December 31, 1991 to D. H. Dolphin et al, from an application Serial No. 455,663 filed December 21, 1989 as a division of application Serial No. 181,859, April 15, 1988, Patent No. 4,892,941, which was a continuation-in-part of Serial No. 39,566, April 17, 1987, abandoned, discloses tetraphenyl porphyrins which are beta-substituted by fluoro or chloro and bear electronegative substituents, for example nitro substituents, on the phenyl.

### DESCRIPTION OF THE INVENTION

We have discovered novel nitro-substituted metalloporphyrins which contain nitro groups in meso and/or beta positions of the porphyrin ring.

The atoms or groups on the meso positions of a metalloporphyrin are represented by the X's in the following structural formula, and the atoms or groups on the β-pyrrolic, or beta, positions by the Y's: 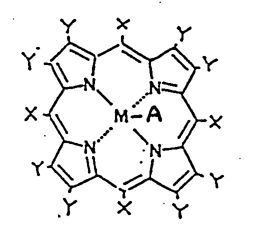
where M is metal, A (1) is an anion such as chloride, bromide, cyanide, azide, nitride, thiocyanate, cyanate, hydroxy, methoxy, chlorate, carboxylates such as acetate, propionate and benzoate, or (2) is absent, said compounds including iron complexes of µ oxo dimers wherein two structures as shown in said formula are joined through an M-O-M linkage.

### COMPLEXES OF CERTAIN METALS WITH MESONITROPORPHYRINS

In one embodiment, the invention is an iron, chromium, ruthenium, manganese, copper or cobalt complex of a metalloporphyrin which has one or more nitro groups, NO₂, in meso positions. Typically, the compound has, in beta positions, either hydrogen atoms, H, or halogen atoms such as fluorine, chlorine or bromine, or nitro or cyano groups, or a hydrocarbon group or a halocarbon group. Examples of halocarbon groups are haloalkyl groups such as perfluoromethyl and perfluoroethyl, and haloaryl groups such as perfluorophenyl. Examples of hydrocarbon groups are aryl groups such as phenyl, substituted phenyl and the like, and alkyl or cycloalkyl groups such as methyl, ethyl and cyclohexyl.

In this embodiment, 1 to 4 of the X's in the above formula are NO₂, 0 to 3 of said X's are hydrogen, halogen, cyano, hydrocarbon or halocarbon, and Y is hydrogen, nitro, cyano, halogen, hydrocarbon or halocarbon. The Y's may all be one atom or group, or different atoms or groups.

The metalloporphyrins of this embodiment differ from the nickel, magnesium or zinc complexes of mesonitrooctaethylporphyrin of the prior art in the metal component of the complex, the nickel, magnesium and zinc complexes being inactive for alkane oxidation, and in some cases by having in beta positions of the porphyrin ring, hydrogen, halogen, halocarbon, cycloalkyl or aryl, rather than the ethyl groups of the prior art compounds.

### METAL COMPLEXES OF BETANITROPORPHYRINS

In another embodiment of the invention, the metalloporphyrin has one or more nitro groups in beta positions and hydrogen or a substituent other than nitro in the remaining beta positions. The substituent may be halo, cyano, hydrocarbon or halocarbon.

In this embodiment, X in the above formula is hydrogen, halogen, nitro, cyano, alkyl, cycloalkyl or halocarbon, at least one of said Y's is nitro, and the remaining Y's are hydrogen, halogen, nitro, cyano, hydrocarbon or halocarbon.

In a preferred embodiment, the compound has either halogen atoms or nitro groups in all of the beta positions. In this embodiment, X in the above formula is hydrogen, nitro, cyano, hydrocarbon or halocarbon, at least one of the Y's is nitro and all of the Y's are either nitro or cyano.

This embodiment differs from the meso-tetraphenyl mono- and di-nitroporphyrins of the Catalano et al article supra in having halogens in meso positions.

Substituents in the meso positions of the metalloporphyrins of this embodiment may be aryl groups such as phenyl, or they may advantageously be perhalocarbon groups such as perfluoromethyl or perfluoroethyl. In this embodiment, X in the above formula is a perhalocarbon group, and Y is hydrogen or nitro, at least one of the Y's being nitro.

This embodiment differs from the meso-tetraphenyl nitroporphyrins of the Catalano et al article in having perhalocarbon groups in meso positions of the porphyrin ring.

In each embodiment of the invention, M in the above formula is preferably Fe, Cr, Mn, Ru, Cu or Co, more preferably Fe.

The compounds of the invention are useful, for example, as catalysts in the oxidation of organic compounds. The manner of usage of the compounds for this purpose is disclosed in applicants copending application, Serial No. 07/758147, filed September 12, 1991.

The terms, porphyrin, porphin and porphine are used interchangeably herein to designate the structure shown in the structural formula supra.

The following examples illustrate the invention:

### Example 1

### Preparation of CuP(NO₂)₄ (Cu mesotetranitroporphin)

0.5 g of CuP (copper porphin) is dissolved in 300 ml of CH₂Cl₂. Through this solution is bubbled NO₂ gas for 5 minutes. (This is an excess - lesser amounts to stoichiometric amounts are also sufficient.) The reaction is stirred at room temperature until the Soret band in the ultraviolet of a sample reaches 427 nm. The material can be purified by column chromatography on a 7.62 by 5.08 cm (3" by 2") neutral alumina column eluting with CHCl₃. The product can also be recrystallized from hot tetrahydrofuran (THF). UV/vis 427, 546, 596 nm (CHCl₃). Mass spectrum shows parent peak at 551.8.

### Example 2

### Preparation of Fe Mesotetranitroporphin Chloride FeP(NO₂)₄Cl

0.25 g of FePCl prepared by the metallation of H₂P with FeCl₂ in THF is dissolved in 200 ml of CH₂Cl₂ and excess NO₂ is bubbled through the solution for 5 minutes. After a few minutes of stirring the Soret band shifts from 396 nm to 428 nm. The CH₂Cl₂ is removed by evaporation and the impure NO₂ complex is purified by column chromatography on neutral alumina or recrystallization in hot THF.

### Example 3

### ZnP(NO₂)₄ (Zn Mesotetranitroporphine)

0.5 g ZnP is dissolved in 300 ml CH₂Cl₂ and excess NO₂ is bubbled through the solution at room temperature for 5 minutes. After stirring for an additional ½ hour the Soret moves from 397 nm to 424 nm. The solvent is removed by evaporation and the solids washed with H₂O. TLC shows a single product UV/VIS (CHCl₃) 424, 521, 571 nm. Infrared (KBr) _{N-O} 1356 cm⁻¹ (strong) and 1541 cm⁻¹ (medium).

### Example 4

### FeP(NO₂)₄Cl from H₂P(NO₂)₄

The meso-tetranitroporphin iron chloride and salts of other metals such as Mn, Co, Cr and Ru can be made by insertion of the metal chloride, acetate or perchlorate salt into H₂P(NO₂)₄ in THF. The H₂P(NO₂)₄ is prepared by removal of Zn from ZnP(NO₂)₄ using 70% HClO₄. 100 mg of ZnP(NO₂)₄ is dissolved in 100 ml of CH₂Cl₂ containing 10 ml of 70% HClO₄. This solution is stirred at room temperature for 1 hour, washed twice with H₂O, then neutralized with saturated sodium bicarbonate aqueous solution. The CH₂Cl₂ is reduced to dryness. The metal, e.g. Fe, is inserted by adding a 10-50% excess of FeCl₂ to a refluxing THF solution of the H₂P(NO₂)₄.

### Example 5

### Preparation of Fe Mesotetranitro β-tetraethyl β-tetratrifluoromethyl Porphin Chloride FeP(NO₂)₄[β(Et)₄-(CF₃)₄]Cl

The complex H₂P[β-(Et)₄₋β-(CF₃)₄] is prepared by condensation of β-ethyl-β-CF₃-2-hydroxymethylpyrrole in acidic media. It can be metallated with FeCl₂ in THF to produce FeP[β-(Et)₄ β-(CF₃)₄]Cl. 0.1 g of this complex is dissolved in 150 ml of CH₂Cl₂ and an excess of NO₂ is bubbled through the solution for 5 minutes producing the tetranitro complex of Fe, FeP(NO₂)₄[β-(Et)₄-β-(CF₃)₄]Cl. The solvent is evaporated, the solid taken up in CHCl₃ and chromatographed on alumina.

## Claims

1. Process for oxidation of alkanes which comprises contacting alkane with oxygen-containing gas in the presence of a metalloporphyrin in which hydrogen atoms in the porphyrin ring have been replaced with at least one nitro group.

2. Process according to claim 1 in which the metalloporphyrin contains iron, chromium, manganese, ruthenium, cobalt or copper.

3. Process according to claim 1 in which 1 to 8 of the pyrrolic hydrogen atoms in the porphyrin ring have been replaced with nitro groups.

4. Process according to claim 3 in which remaining hydrogen atoms in-the porphyrin ring have been substituted with halogen atoms.

5. Process according to claim 4 in which 4 to 8 hydrogen atoms in the porphyrin ring have been replaced with nitro groups and in which 8 to 20 hydrogen atoms in the porphyrin ring have been replaced with halogen.

6. Process according to claim 1 in which the metalloporphyrin is a nitrated, mesoperfluorinated alkyl-porphyrin.

7. Process according to claim 1 in which the metalloporphyrin is a nitrated iron tetrakispentafluorophenylporphine.

8. Process according to claim 1 wherein the metalloporphyrin is a metallomesotetranitroporphine.

9. A compound having the formula: 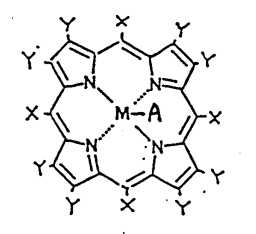 where M is iron, chromium, manganese, ruthenium, copper or cobalt, 1 to 4 of said X's are nitro, 0 to 3 of said X's are hydrogen, halogen, cyano, hydrocarbon or halocarbon, Y is hydrogen, halogen, nitro, cyano, hydrocarbon or halocarbon, is either (1) an anion such as chloride, bromide, fluoride, cyanide, azide, nitride, thiocyanate, cyanate, hydroxy, methoxy, chlorate, carboxylate, or (2) is absent, said compounds including iron complexes of µ oxo dimers comprising two structures as shown in said formula joined through an M-O-M linkage.

10. Compound according to claim 9 wherein Y is hydrogen, halogen, nitro, cyano, halocarbon, aryl or cycloalkyl.

11. according to claim 9 wherein each X is nitro.

12. Compound according to claim 11 wherein M is copper and each Y is hydrogen.

13. is an iron complex of mesotetranitroporphyrin or mesotetranitroporphyrin halide.

14. according to claim 9, which is a copper complex of mesotetranitroporphyrin.

15. a zinc complex of mesotetranitroporphyrin.

16. an iron complex of mesotetranitro β-tetraethyl-β-tetra (trifluoromethyl)-porphyrin halide.

17. according to claim 9 wherein A is chloride, bromide, fluoride, hydroxy or azide.

18. according to claim 9 wherein said compound is said iron complex of µ oxo dimer.

19. A compound having the formula: 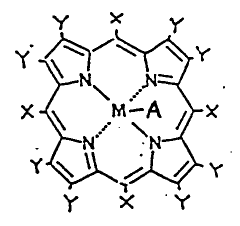 where M is iron, chromium, manganese, ruthenium, copper or cobalt, X is hydrogen, halogen, nitro, cyano, alkyl, cycloalkyl or halocarbon, at least one of said Y's is nitro the remaining Y's are hydrogen, halogen, nitro, cyano, hydrocarbon or halocarbon, A is either (1) an anion such as chloride, bromide, fluoride, cyanide, azide, nitride, thiocyanate, cyanate, hydroxy, methoxy, chlorate, carboxylate, or (2) is absent.

20. according to claim 19 wherein said remaining Y's are halogen.

21. according to claim 19 wherein each X is fluorocarbon.

22. Compound according to claim 19, which is an iron complex of mesotetraperfluoroalkyl-β-nitroporphyrin, having 1 to 7 carbon atom in said alkyl group.

23. according to claim 19 wherein A is chloride, bromide, fluoride, hydroxy or azide.

24. according to claim 19 wherein said compound is said iron complex of µ oxo dimer.

## Patentansprüche

1. Verfahren zur Oxidation von Alkanen, welches das Kontaktieren eines Alkans mit sauerstoffhaltigem Gas in Gegenwart eines Metallporphyrins, in welchem Wasserstoffatome im Porphyrinring durch mindestens eine Nitrogruppe ersetzt worden sind, umfaßt.

2. Verfahren gemäß Anspruch 1, wobei das Metallporphyrin Eisen, Chrom, Mangan, Ruthenium, Cobalt oder Kupfer enthält.

3. Verfahren gemäß Anspruch 1, wobei 1 bis 8 der Pyrrolwasserstoffatome im Porphyrinring durch Nitrogruppen ersetzt worden sind.

4. Verfahren gemäß Anspruch 3, wobei die übrigen Wasserstoffatome im Porphyrinring durch Halogenatome ersetzt worden sind.

5. Verfahren gemäß Anspruch 4, wobei 4 bis 8 Wasserstoffatome im Porphyrinring durch Nitrogruppen ersetzt worden sind und wobei 8 bis 20 Wasserstoffatome im Porphyrinring durch Halogene ersetzt worden sind.

6. Verfahren gemäß Anspruch 1, wobei das Metallporphyrin eine nitriertes, mesoperfluoriertes Alkylporphyrin ist.

7. Verfahren gemäß Anspruch 1, wobei das Metallporphyrin ein nitriertes Eisentetrakispentafluorphenylporphin ist.

8. Verfahren gemäß Anspruch 1, wobei das Metallporphyrin ein Metallomesotetranitroporphin ist.

9. Verbindung der Formel: 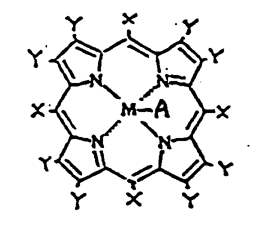 worin M Eisen, Chrom, Mangan, Ruthenium, Kupfer oder Cobalt ist, 1 bis 4 der Substituenten X Nitro sind, 0 bis 3 der Substituenten X Wasserstoff, Halogen, Cyan, Kohlenwasserstoff oder Halogenkohlenstoff sind, Y Wasserstoff, Halogen, Nitro, Cyan, Kohlenwasserstoff oder Halogenkohlenstoff ist, A entweder (1) ein Anion, zum Beispiel Chlorid, Bromid, Fluorid, Cyanid, Azid, Nitrid, Thiocyanat, Cyanat, Hydroxy, Methoxy, Chlorat oder Carboxylat, ist oder (2) nicht vorhanden ist, wobei die Verbindung Eisenkomplexe aus µ-Oxodimeren umfaßt, welche zwei der in der Formel gezeigten Strukturen enthält, die durch eine M-O-M Bindung verknüpft sind.

10. Verbindung gemäß Anspruch 9, worin Y Wasserstoff, Halogen, Nitro, Cyan, Halogenkohlenstoff, Aryl oder Cycloalkyl ist.

11. Verbindung gemäß Anspruch 9, worin jedes X eine Nitrogruppe ist.

12. Verbindung gemäß Anspruch 11, worin M Kupfer ist und jedes Y Wasserstoff ist.

13. Verbindung gemäß Anspruch 9, die ein Eisenkomplex aus Mesotetranitroporphyrin oder Mesotetranitroporphyrinhalogenid ist.

14. Verbindung gemäß Anspruch 9, die ein Kupferkomplex aus Mesotetranitroporphyrin ist.

15. Verbindung gemäß Anspruch 9, die ein Zinkkomplex aus Mesotetranitroporphyrin ist.

16. Verbindung gemäß Anspruch 9, die ein Eisenkomplex aus Mesotetranitro-β-tetraethyl-β-tetra(trifluormethyl)porphyrinhalogenid ist.

17. Verbindung gemäß Anspruch 9, worin A Chlorid, Bromid, Fluorid, Hydroxy oder Azid ist.

18. Verbindung gemäß Anspruch 9, worin die Verbindung der Eisenkomplex eines µ-Oxodimeren ist.

19. Verbindung der Formel: 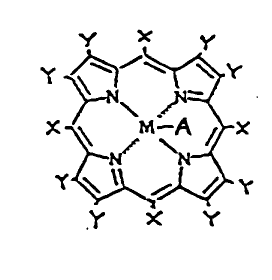 worin M Eisen, Chrom, Mangan, Ruthenium, Kupfer oder Cobalt ist, X Wasserstoff, Halogen, Nitro, Cyan, Alkyl, Cycloalkyl oder Halogenkohlenstoff ist, mindestens ein Y Nitro ist und die übrigen Substituenten Y gleich Wasserstoff, Halogen, Nitro, Cyan, Kohlenwasserstoff oder Halogenkohlenstoff sind, A entweder (1) ein Anion, zum Beispiel Chlorid, Bromid, Fluorid, Cyanid, Azid, Nitrid, Thiocyanat, Cyanat, Hydroxy, Methoxy oder Carboxylat, ist oder (2) nicht vorhanden ist.

20. Verbindung gemäß Anspruch 19, worin die übrigen Substituenten Y Halogen sind.

21. Verbindung gemäß Anspruch 19, worin jedes X Fluorkohlenstoff ist.

22. Verbindung gemäß Anspruch 19, die ein Eisenkomplex aus Mesotetraperfluoralkyl-β-nitroporphyrin ist, wobei die Alkylgruppe 1 bis 7 Kohlenstoffatome aufweist.

23. Verbindung gemäß Anspruch 19, worin A Chlorid, Bromid, Fluorid, Hydroxy oder Azid ist.

24. Verbindung gemäß Anspruch 19, worin die Verbindung der Eisenkomplex eines µ-Oxodimeren ist.

## Revendications

1. Procédé d'oxydation d'alcanes, caractérisé en ce que l'on met un alcane en contact avec un gaz contenant de l'oxygène, en présence d'une métalloporphyrine dans laquelle des atomes d'hydrogène du noyau porphyrine ont été remplacés par au moins un groupe nitro.

2. Procédé suivant la revendication 1, caractérisé en ce que la métalloporphyrine contient du fer, du chrome, du manganèse, du ruthénium, du cobalt ou du cuivre.

3. Procédé suivant la revendication 1, caractérisé en ce que de 1 à 8 des atomes d'hydrogène pyrroliques du noyau porphyrine ont été remplacés par des groupes nitro.

4. Procédé suivant la revendication 3, caractérisé en ce que les atomes d'hydrogène résiduels du noyau porphyrine ont été remplacés par des atomes d'halogènes.

5. Procédé suivant la revendication 4, caractérisé en ce que de 4 à 8 atomes d'hydrogène du noyau porphyrine ont été remplacés par des groupes nitro et en ce que 8 à 20 atomes d'hydrogène du noyau porphyrine ont été remplacés par des atomes d'halogènes.

6. Procédé suivant la revendication 1, caractérisé en ce que la métalloporphyrine est une alkylporphyrine nitrée, mésoperfluorée.

7. Procédé suivant la revendication 1, caractérisé en ce que la métalloporphyrine est une tétrakis-pentafluorophénylporphyrine de fer, nitrée.

8. Procédé suivant la revendication 1, caractérisé en ce que la métalloporphyrine est une métallomésotétranitroporphine.

9. Composé répondant à la formule suivante : 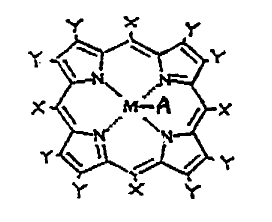 dans laquelle M représente le fer, le chrome, le manganèse, le ruthénium, le cuivre ou le cobalt, de 1 à 4 des symboles X sont des radicaux nitro, de 0 à 3 des symboles X représentent des atomes d'hydrogène, des atomes d'halogènes, des radicaux cyano, des restes d'hydrocarbures ou d'hydrocarbures halogénés, Y représente un atome d'hydrogène, un atome d'halogène, un radical nitro, cyano, un reste d'hydrocarbure ou d'hydrocarbure halogéné, A représente (1) un anion tel qu'un anion chlorure, bromure, fluorure, cyanure, azide, nitrure, thiocyanate, cyanate, hydroxyle, méthoxy, chlorate, carboxylate, ou (2) est absent, ledit composé comprenant des complexes du fer de µ-oxodimères comprenant deux structures telles que représentées dans ladite formule, unies par l'intermédiaire d'une liaison M-O-M.

10. Composé suivant la revendication 9, caractérisé en ce que Y représente un atome d'hydrogène, un atome d'halogène, un radical nitro, cyano, un reste d'hydrocarbure halogéné, un radical aryle ou cycloalkyle.

11. Composé suivant la revendication 9, caractérisé en ce que chaque symbole X représente un radical nitro.

12. Composé suivant la revendication 11, caractérisé en ce que M représente le cuivre et chaque symbole Y représente un atome d'hydrogène.

13. Composé suivant la revendication 9, caractérisé en ce qu'il est un complexe du fer de la mésotétranitroporphyrine ou d'un halogénure de mésotétranitroporphyrine.

14. Composé suivant la revendication 9, caractérisé en ce qu'il est un complexe du cuivre de la mésotétranitroporphyrine.

15. Composé suivant la revendication 9, caractérisé en ce qu'il est un complexe du zinc de la mésotétranitroporphyrine.

16. Composé suivant la revendication 9, caractérisé en ce qu'il est un complexe du fer d'un halogénure de mésotétranitro-β-tétraéthyl-β-tétra-(trifluorométhyl)-porphyrine.

17. Composé suivant la revendication 9, caractérisé en ce que A représente un anion chlorure, bromure, fluorure, hydroxyle ou azide.

18. Composé suivant la revendication 9, caractérisé en ce qu'il est un complexe du fer d'un µ-oxodimère.

19. Composé répondant à la formule suivante : 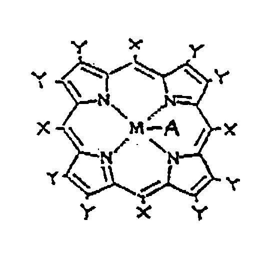 dans laquelle M représente le fer, le chrome, le manganèse, le ruthénium, le cuivre ou le cobalt, X représente un atome d'hydrogène, un atome d'halogène, un radical nitro, cyano, alkyle, cycloalkyle ou un reste d'hydrocarbure halogéné, au moins l'un des symboles Y est un radical nitro, cependant que les autres symboles Y représentent des atomes d'hydrogène, des atomes d'halogènes, des radicaux nitro, cyano, des restes d'hydrocarbures ou d'hydrocarbures halogénés, A est un anion, tel qu'un anion chlorure, bromure, fluorure, cyanure, azide, nitrure, thiocyanate, cyanate, hydroxyle, méthoxy, chlorate, carboxylate, ou (2) est absent.

20. Composé suivant la revendication 19, caractérisé en ce que les autres symboles Y représentent des atomes d'halogènes.

21. Composé suivant la revendication 19, caractérisé en ce que chaque symbole X représente un reste d'hydrocarbure fluoré.

22. Composé suivant la revendication 19, caractérisé en ce qu'il est un complexe du fer de la mésotétraperfluoralkyl-β-nitroporphyrine, dont le radical alkyle comporte de 1 à 7 atomes de carbone.

23. Composé suivant la revendication 19, caractérisé en ce que A représente un anion chlorure, bromure, fluorure, hydroxyle ou azide.

24. Composé suivant la revendication 19, caractérisé en ce qu'il est un complexe du fer d'un µ-oxodimère.
